# EUROPÄISCHE PATENTANMELDUNG

(11) **EP 0 704 866 A1**
(43) Veröffentlichungstag der Anmeldung: **03.04.1996**
(21) Anmeldenummer: 95115471.5
(22) Anmeldetag: 29.09.1995
(51) Int. Cl.: H01F 27/40, H02H 5/08, G01N 33/28

(54) **Transformator**

(30) Priorität: 30.09.1994 DE 4435055
(71) Anmelder: SIEMENS AKTIENGESELLSCHAFT, D-80333 München (DE)
(72) Erfinder: Alber, Friedrich, Dipl.-Ing. (FH), D-73257 Köngen (DE); Koch, Hans-Günther, D-71397 Leutenbach (DE); Böhme, Jens-Dieter, Dipl.-Ing. (FH), D-73230 Kirchheim (DE); Ritter, Herbert, Dipl.-Ing. (FH), D-73054 Eislingen (DE); Buck, Robert, D-73230 Kirchheim (DE)

(57) **Zusammenfassung**

Um eine einfache Auslösung bei der Bildung von Gasblasen in einem ölgefüllten Kessel (2) eines Transformators (1) zu erzielen, ist eine Sicherheitseinrichtung (15) vorgesehen, die Elektroden (17x,17y,17z) umfaßt. Die Elektroden (17x,17y, 17z) sind elektrisch mit den Phasenwicklungen (5x,5y,5z) verbunden, wobei ihr Abstand derart bemessen ist, daß die anliegende Spannung höher als die Durchschlagsfestigkeit in Luft oder Gas und kleiner als im Öl ist. Beim Auftreten eines Überschlags an den Elektroden (17x,17y,17z) löst eine zugeordnete Schutzvorrichtung, insbesondere Sicherungen (11x,11y, 11z) aus.

## Beschreibung

Die Erfindung betrifft einen Transformator mit einem Isoliermedium gefüllten Kessel, in dem ein Kern mit Phasenwicklungen angeordnet ist und einer im Kessel zumindest teilweise im Isoliermedium angeordneten Sicherheitseinrichtung.

Zum Schutz von Transformatoren in der Hoch- und Mittelspannungstechnik ist es bekannt, Schutzeinrichtungen vorzusehen, die im ölgefüllten Kessel des Transformators angeordnet sind. Dies betrifft beispielsweise thermische Schutzeinrichtungen zur Überwachung von Übertemperatur oder mangelhafte Kühlung oder das sogenannte Buchholzrelais gegen Ölverlust oder Gasbildung durch schleichende Fehler und Ölschwall. Darüber hinaus sind auch elektrische Schutzeinrichtungen vorgesehen, die den Transformator auf Überströme oder Überlast (Sicherungen oder Überstromzeitschutz) überwachen.

Die erstgenannten Schutzeinrichtungen können den Transformator nicht selbst vom Netz trennen, sondern wirken über Steuersignale auf dem Transformator vorgeschalteten Schalter und benötigen daher zusätzliche Steuereinrichtungen die aufwendig sind.

Der Erfindung liegt die Aufgabe zugrunde, einen Transformator mit einer einfachen Sicherheitseinrichtung für seine Kühlmitteltemperatur und/oder seinen Kühlmittelstand anzugeben, bei der zusätzliche Steuereinrichtungen oder Verbindungsleitungen zur Signalisierung und/oder Auslösung nicht benötigt werden.

Die Aufgabe wird erfindungsgemäß gelöst mit einem Transformator mit
- einem mit einem Isoliermedium gefüllten Kessel, in dem ein Kern mit Phasenwicklungen angeordnet ist und
- einer im Kessel zumindest teilweise im Isoliermedium angeordneten Sicherheitseinrichtung, wobei
- die Sicherheitseinrichtung mit einer Spannung versorgte Elektroden aufweist, die in einem vorgegebenen Abstand voneinander angeordnet sind,
- wobei der Abstand der Elektroden derart bemessen ist, daß die anliegende Spannung höher als die Durchschlagsfestigkeit in Luft oder Gas und kleiner als in Öl ist und
- die Elektroden mit einer Schutzvorrichtung in Wirkverbindung stehen, derart, daß bei einem Überschreiten der Überschlagsfestigkeit eine Auslösung der Sicherheitsvorrichtung erfolgt,
Auf diese einfache Weise kann ein Ölstand oder eine Gasbildung ohne zusätzliche Steuereinrichtungen sicher überwacht werden. Im Transformator sind dabei nur einfache wartungsfreie Bauteile angeordnet, die zuverlässig arbeiten.

Mit Vorteil sind die freien Enden der Elektroden in einem oberen Bereich des Kessels angeordnet und bilden Eckpunkte eines regelmäßigen Vielecks. Damit ist eine gute Auslösung beim Auftreten von Gasblasen möglich.

Bevorzugt sind die freien Enden der Elektroden in einem am Kessel nach oben gerichteten Dom angeordnet. Damit ist eine Sammlung von Gasblasen möglich, wodurch eine schnelle und frühzeitige Abschaltung im Fehlerfall erfolgen kann.

Mit Vorteil sind die Elektroden jeweils mit einer der Wicklungen des Transformators galvanisch verbunden. Es wird also keine zusätzliche Energie für die Sicherheitseinrichtung benötigt. Der Aufbau gestaltet sich daher sehr einfach.

Die Anzahl der Elektroden kann gleich der Anzahl von Phasen sein. Damit ist ein symmetrischer Aufbau gegeben, wobei auch beim Ausfall einer der Phasenspannungen noch eine Auslösung gewährleistet ist.

Zusätzlich können auch Mittel zur Erniedrigung der Durchschlagsfestigkeit vorgesehen sein. Dadurch ist eine weitere oder redundante Nutzung der vorhandenen Mittel zur Fehlererfassung oder zur Auslösung im Störfall gegeben.

Die Mittel können einen Kurzschlußstößel umfassen, der die freien Enden der Elektroden kurzschließt. Damit ist eine einfache Lösung gegeben, die sehr effektiv und zuverlässig arbeitet. Der Kurzschlußstößel kann dabei mit Feder- und/oder Schwerkraft verschiebbar sein, wobei eine Anpassung an die jeweiligen Bedingungen gegeben ist.

Bevorzugt steht die Bewegungsachse des Kurzschlußstößels senkrecht auf der Ebene des von den Elektroden gebildeten Vielecks. Somit ist eine gleichmäßige Auslösung im Stör- oder Fehlerfall für alle Phasen gegeben.

Der Kurzschlußstößel kann im Normalbetrieb des Transformators durch ein Bimetall verriegelt sein, das den Kurzschlußstößel beim Erreichen einer voreingestellten Temperatur freigibt. Damit ist eine zusätzliche Auslösung bei Übertemperatur möglich.

Mit Vorteil ist die Schutzvorrichtung von den Sicherungen der jeweiligen Phasen gebildet. Damit ist eine Nutzung von ohnehin vorhandenen Mitteln möglich, wobei kein zusätzlicher Aufwand entsteht.

Die Schutzvorrichtung kann auch einen Transformatordiffschutz, einem Impedanzschutz, einen Erdschlußschutz oder eine sonstige Netzschutzeinrichtung umfassen. Dadurch kann der Transformator in ein bestehendes Schutzkonzept, gegebenenfalls mit allen signaltechnischen Vorzügen, eingebunden werden, ohne daß zusätzlicher Aufwand entsteht.

In einer weiteren Ausführung können die Mittel ein Bimetall umfassen, das beim Erreichen einer vorgegebenen Temperatur einen Überschlag an den Elektroden erzeugt. Dadurch ist eine besonders einfache Ausbildung für die Erfassung von Übertemperaturen gegeben.

Die Erfindung, weitere Details und Vorteile werden nachfolgend anhand der Zeichnung beispielhaft näher erläutert. Es zeigen:
- FIG 1: einen Transformator mit der neuen Sicherheitseinrichtung und
- FIG 2: die Sicherheitseinrichtung im Detail.

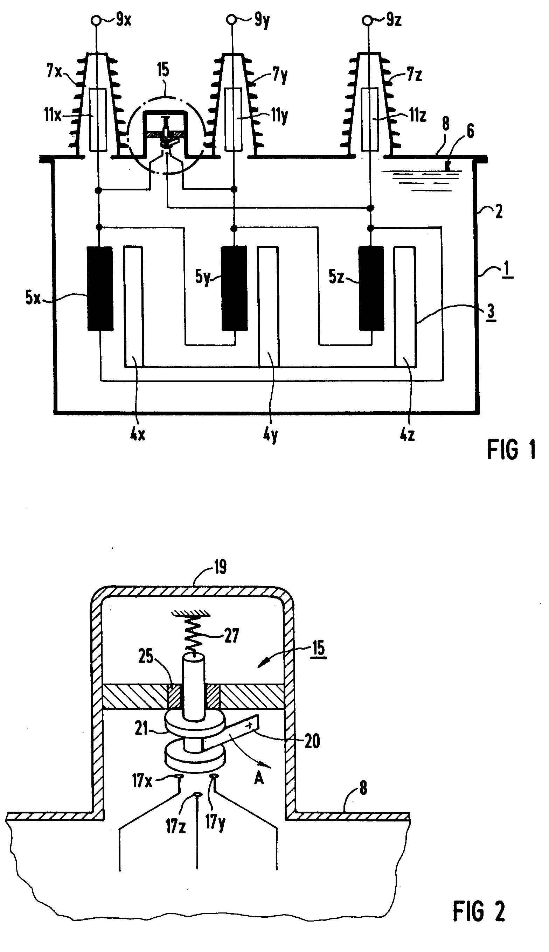

In FIG 1 ist ein Transformator 1 in einer Prinzipdarstellung gezeigt. In seinem Kessel 2 ist dabei ein Kern 3 angeordnet. Der Kern 3 trägt auf seinen Schenkeln 4x, 4y und 4z jeweils eine Phasenwicklung 5x bzw. 5y bzw. 5z. Diese sind vorliegend als Oberspannungswicklungen ausgeführt. Die zugeordneten Unterspannungswicklungen sind nicht näher dargestellt. Die Phasenwicklungen 5x, 5y und 5z sind dabei in Dreieck geschaltet. Der Kessel 2 ist mit einem Isoliermedium 6, bevorzugt Öl, gefüllt.

Der Kessel 2 weist an seiner oberen Seite einen Kesseldeckel 8 auf, an dem je Phase eine Durchführung 7x bzw. 7y bzw. 7z angeordnet ist. Über diese Durchführungen 7x, 7y und 7z sind die Anschlüsse der Phasenwicklungen 5x, 5y und 5z nach außen an jeweilige Klemmen 9x, 9y und 9z geführt. Die Klemmen 9x, 9y und 9z dienen zum oberspannungsseitigen Anschluß des Transformators 1.

Zwischen den Phasenwicklungen 5x, 5y und 5z und ihren jeweiligen Klemmen 9x, 9y und 9z ist jeweils als Schutzvorrichtung eine Sicherung 11x bzw. 11y bzw. 11z geschaltet. Die Sicherungen 11x, 11y und 11z können beispielsweise als Schmelzsicherungen zur Begrenzung eine maximalen Stromes dienen.

Zur zusätzlichen Überwachung weist der Transformator 1 eine im Öl befindliche Sicherheitseinrichtung 15 auf, die bevorzugt in einem oberem Bereich des Kessels 2, insbesondere in einem Dom 19 angeordnet ist. Der Dom 19 begünstigt eine Sammlung von entstehenden Gasblasen. Die Sicherheitseinrichtung 15 ist dabei in FIG 2 im Detail dargestellt.

Die Sicherheitseinrichtung 15 weist zunächst je Phase des Transformators 1 eine Elektrode 17x, 17y und 17z auf, deren freie Enden bevorzugt Eckpunkte eines regelmäßigen Vielecks bilden. Die Anzahl der Elektroden kann gegebenenfalls auch geringer als die Phasenanzahl sein.

Der Abstand zwischen den Elektroden ist derart bemessen, daß die jeweils anliegende Spannung höher als die Durchschlagsfestigkeit in Luft oder Gas und kleiner als in Öl ist. Dadurch ist erzielt, daß im normalen Betrieb kein Überschlag stattfindet. Sammeln sich jedoch während des Betriebs Gasblasen im Bereich der Elektroden 17x, 17y und 17z an, so findet ein Überschlag statt, der einen Kurzschluß zwischen den Phasenwicklungen 5x, 5y und 5z entspricht. Es tritt dabei ein Kurzschlußstrom auf, so daß die jeweils betroffenen Sicherungen 11x, 11y und 11z ansprechen und auslösen. Gegebenenfalls kann auch eine Reaktion oder eine Auslösung von einem nicht näher dargestellten Schutzgerät erfolgen, z.B. von einem Trafodiffschutz oder einem Differenzialschutz.

Gegebenenfalls können auch zusätzliche Mittel zur Erniedrigung der Durchschlagsfestigkeit zwischen den Elektroden 17x, 17y und 17z vorgesehen sein. Im einfachsten Fall kann hierzu ein einfaches Bimetall dienen, das nach einer vorgegebenen Erwärmung sich in den Bereich der Elektroden bewegt und somit die Durchschlagsfestigkeit dort erniedrigt. Die Folge ist ein Ansprechen der in Wirkverbindung stehenden Sicherheitsvorrichtung.

In der FIG 2 ist als Mittel ein mittels einer Feder 20 verschiebbarer Kurzschlußschlüssel 21 vorgesehen, dessen Bewegungsrichtung senkrecht auf der Ebene des von den Elektroden 17x, 17y und 17z gebildeten Vielecks steht.

Bei einer temperaturbedingten Bewegung des Bimetalls 20 in Pfeilrichtung A wird der Kurzschlußstößel 21 in Richtung der Elektroden 17x, 17y und 17z freigegeben, wodurch im einfachsten Fall die Durchschlagsfestigkeit erniedrigt wird. Gegebenenfalls kann der Kurzschlußstößel 21 auch die Elektroden 17x, 17y und 17z durch Kontaktierung kurzschließen.

Der Antrieb des Kurzschlußstößel 21 kann bei Bedarf auch mittels Schwerkraft erfolgen. Er kann gegebenenfalls auch einen gasgefüllten Hohlraum umfassen, der nach Art eines Füllstandmelders in Abhängigkeit vom Ölstand innerhalb des Kessels 2 betätigbar ist. Der Kurzschlußstößel 21 ist in seiner Bewegungsrichtung beispielhaft durch ein Lager 25 geführt.

## Patentansprüche

1. Transformator (1) mit
- einem mit einem Isoliermedium gefüllten Kessel (2), in dem ein Kern (3) mit Phasenwicklungen (5x,5y,5z) angeordnet ist und
- einer im Kessel (2) zumindest teilweise im Isoliermedium angeordneten Sicherheitseinrichtung (15),
**dadurch gekennzeichnet,** daß
- die Sicherheitseinrichtung (15) mit einer Spannung versorgte Elektroden (17x,17y,17z) aufweist, die in einem vorgegebenen Abstand voneinander angeordnet sind,
- wobei der Abstand der Elektroden (17x,17y,17z) derart bemessen ist, daß die anliegende Spannung höher als die Durchschlagsfestigkeit in Luft oder Gas und kleiner als in Öl ist und
- die Elektroden (17x,17y,17z) mit einer Schutzvorrichtung in Wirkverbindung stehen, derart, daß bei einem Überschreiten der Überschlagsfestigkeit zwischen den Elektroden (17x,17y, 17z) eine Auslösung der Sicherheitsvorrichtung (15) erfolgt.

2. Transformator nach Anspruch 1, **dadurch gekennzeichnet,** daß die freien Enden der Elektroden (17x,17y,17z) in einem oberen Bereich des Kessels (2) angeordnet sind.

3. Transformator nach Anspruch 1 oder 2, **dadurch gekennzeichnet,** daß die freien Enden der Elektroden (17x,17y,17z) Eckpunkte eines regelmäßigen Vielecks bilden.

4. Transformator nach Anspruch 1, 2 oder 3, **dadurch gekennzeichnet,** daß die freien Enden der Elektroden (17x,17y,17z) in einem am Kessel (2) nach oben gerichteten Dom (19) angeordnet sind.

5. Transformator nach einem der Ansprüche 1 bis 4, **dadurch gekennzeichnet,** daß die Elektroden (17x,17y,17z) jeweils mit einer der Phasenwicklungen (5x,5y,5z) galvanisch verbunden sind.

6. Transformator nach einem der Ansprüche 1 bis 5, **dadurch gekennzeichnet,** daß die Anzahl der Elektroden (17x,17y,17z) gleich der Anzahl von Phasenwicklungen (5x,5y,5z) ist.

7. Transformator nach einem der Ansprüche 1 bis 6, **dadurch gekennzeichnet,** daß Mittel zur Erniedrigung der Durchschlagsfestigkeit vorgesehen sind.

8. Transformator nach Anspruch 7, **dadurch gekennzeichnet,** daß die Mittel einen Kurzschlußstößel (21) umfassen, der die freien Enden der Elektroden (17x,17y,17z) kurzschließt.

9. Transformator nach Anspruch 8, **dadurch gekennzeichnet,** daß der Kurzschlußstößel (21) mit Feder- und/oder Schwerkraft verschiebbar ist.

10. Transformator nach Anspruch 9, **dadurch gekennzeichnet,** daß die Bewegungsachse des Kurzschlußstößels (21) senkrecht auf der Ebene des von den Elektroden (17x,17y,17z) gebildeten Vielecks steht.

11. Transformator nach einem der Ansprüche 8 bis 10, **dadurch gekennzeichnet,** daß der Kurzschlußstößel (21) im Normalbetrieb durch ein Bimetall (20) verriegelt ist, das den Kurzschlußstößel (21) beim Erreichen einer voreingestellten Temperatur freigibt.

12. Transformator nach einem der Ansprüche 1 bis 11, **dadurch gekennzeichnet,** daß die Schutzvorrichtung von den Sicherungen (11x,11y,11z) der jeweiligen Phasen gebildet ist.

13. Transformator nach einem der Ansprüche 1 bis 11, **dadurch gekennzeichnet,** daß die Schutzvorrichtung einen Transformatordiffschutz, einem Impedanzschutz, einen Erdschlußschutz oder eine sonstige Netzschutzeinrichtung umfaßt.

14. Transformator nach Anspruch 7, **dadurch gekennzeichnet,** daß die Mittel ein Bimetall umfassen, das beim Erreichen einer vorgegebenen Temperatur einen Überschlag an den Elektroden (17x,17y,17z) erzeugt.

15. Transformator nach einem der Ansprüche 1 bis 14, **dadurch gekennzeichnet,** daß das Isoliermedium Öl ist.
